# EUROPEAN PATENT SPECIFICATION

(11) **EP 3 289 084 B1**
(45) Date of publication and mention of the grant of the patent: **16.09.2020**
(21) Application number: 16780820.3
(22) Date of filing: 15.04.2016
(51) Int. Cl.: C12N 15/113, A61K 31/713, A61P 35/00

(54) **siRNA INHIBITION OF HUMAN ANTIGEN R EXPRESSION FOR TREATMENT OF CANCER**
SIRNA-HEMMUNG DER EXPRESSION DES MENSCHLICHEN ANTIGENS R ZUR BEHANDLUNG VON KREBS
INHIBITION PAR ARNSI DE L'EXPRESSION DE L'ANTIGÈNE R HUMAIN POUR LE TRAITEMENT DU CANCER

(30) Priority: 17.04.2015 US 201562148869 P
(43) Date of publication of application: 07.03.2018
(73) Proprietor: Genisphere, LLC, Hatfield, PA 19440 (US); Lankenau Institute for Medical Research, Wynnewood, PA 19096 (US)
(72) Inventor: GETTS, Robert C., Collegeville, Pennsylvania 19426 (US); SAWICKI, Janet, Newtown Square, Pennsylvania 19073 (US)
(74) Representative: FRKelly
(86) International application number: PCT/US2016/027723
(87) International publication number: WO 2016/168578

(56) References cited:
- WO-A1-2014/121050
- WO-A1-2014/121050
- US-A1- 2012 122 800
- R MURALIDHARAN: "Abstract 5418: Tumor-targeted nanoparticle delivery of HuR-RNAi suppresses lung cancer cell proliferation and cell migration", CANCER RESEARCH, vol. 74, no. 19, 1 January 2014 (2014-01-01), XP055322479, DOI: 10.1158/1538-7445.AM2014-5418
- JALEH VARSHOSAZ ET AL: "Nanoparticles for targeted delivery of therapeutics and small interfering RNAs in hepatocellular carcinoma", WORLD JOURNAL OF GASTROENTEROLOGY, vol. 21, no. 42, 1 January 2015 (2015-01-01), pages 12022-21, XP55512735, CN ISSN: 1007-9327, DOI: 10.3748/wjg.v21.i42.12022
- YU-HUNG HUANG ET AL: "Delivery of Therapeutics Targeting the mRNA-Binding Protein HuR Using 3DNA Nanocarriers Suppresses Ovarian Tumor Growth", CANCER RESEARCH, vol. 76, no. 6, 26 February 2016 (2016-02-26), pages 1549-1559, XP55512743, US ISSN: 0008-5472, DOI: 10.1158/0008-5472.CAN-15-2073
- DATABASE NCBI [Online] 12 March 2015 'Homo sapiens ELAV like RNA binding protein 1 (ELAVL1), transcript variant X1, mRNA`', XP055322482 Database accession no. XM_011527777.1
- PARK, J. H. ET AL.: 'Effect of siRNA with an asymmetric RNA/dTdT overhang on RNA interference activity' NUCLEIC ACID THERAPEUTICS vol. 24, no. 5, October 2014, pages 364 - 371, XP055322414
- DATABASE NCBI [Online] 15 March 2015 NCBI: 'Homo sapiens ELAV like RNA binding protein 1 (ELAVL1), mRNA', XP055322482 Database accession no. NM_001419.2
- MURALIDHARAN, R. ET AL.: 'Tumor-targeted nanoparticle delivery of HuR-RNAi suppresses lung cancer cell proliferation and cell migration' CANCER RESEARCH vol. 74, no. 19, 2014, XP055322479
- AL-AHMADI, W. ET AL.: 'miR-29a inhibition normalizes HuR over-expression and aberrant AU -rich mRNA stability in invasive cancer' J. PATHOL. vol. 230, no. 1, May 2013, pages 1 - 11, XP055322417

### SEQUENCE LISTING

<110> Genisphere, LLC Lankenau Institute of Medical Research Getts, Robert C. Sawicki, Janet
<120> siRNA Inhibition of Human Antigen R Expression for Treatment of Cancer
<130> DSC0058-00WO
<150> 62/148,869
   <151> 2015-04-17
<160> 4
<170> PatentIn version 3.5
<210> 1
   <211> 21
   <212> DNA
   <213> Mouse
<220>
   <221> misc_feature
   <222> (1)..(19)
   <223> ribonucleotides
<220>
   <221> misc_feature
   <222> (20)..(21)
   <223> deoxyribonucleotides
<400> 1
   gccuguucag cagcauuggt t 21
<210> 2
   <211> 21
   <212> DNA
   <213> Human
<220>
   <221> misc_feature
   <222> (1)..(19)
   <223> ribonucleotides
<220>
   <221> misc_feature
   <222> (20)..(21)
   <223> deoxyribonucleotides
<400> 2
   gcguuuaucc gguuugacat t 21
<210> 3
   <211> 21
   <212> DNA
   <213> Mouse
<220>
   <221> misc_feature
   <222> (1)..(19)
   <223> ribonucleotides
<220>
   <221> misc_feature
   <222> (20)..(21)
   <223> deoxyribonucleotides
<400> 3
   ccaaugcugc ugaacaggct t 21
<210> 4
   <211> 21
   <212> RNA
   <213> Human
<220>
   <221> misc_feature
   <222> (20)..(21)
   <223> Dicer processing
<400> 4
   ugucaaaccg gauaaacgca a 21

## Description

### TECHNICAL FIELD

Principles and embodiments of the present description relate generally to targeted DNA dendrimer delivery of siRNA to inhibit expression of Human antigen R (HuR or ELAVL1) and compositions useful for inhibition of HuR expression.

### BACKGROUND

Human antigen R (also known as HuR or ELAVL1) is a post-transcriptional regulatory RNA binding protein which regulates the expression of multiple genes involved in tumor cell survival and drug resistance. mRNA stability protein encoded by the *ELAV1* gene. It is abundant and hyper-functional in ovarian cancer, and is also highly expressed in breast cancer, lung cancer, mesothelioma and colon cancer.

The cells of certain tumors express elevated levels of receptors for folic acid (folate) on their surfaces. This allows for delivery of therapeutics specifically to such tumors following systemic administration, as folate-drug conjugates bind to the folate receptor and trigger cellular uptake by endocytosis. Among the tumors expressing elevated levels of folate receptors are ovarian cancers, mesothelioma, breast cancer, colon cancer, and lung cancer.

DNA dendrimers are highly branched molecules constructed from partially double-stranded DNA monomer subunits, each of which comprises two single-stranded DNA molecules that share a region of sequence complementarity located in the central portion of each strand but are non-complementary at the 5' and 3' ends. The single-stranded ends of the monomers form four "arms" that are available for hybridization to a complementary arm sequence on another monomer. The DNA dendrimer is thus built in layers by hybridization of different complementary single-stranded arms. Available unhybridized arms may be used for attachment of a cargo, such as a targeting moiety, a therapeutic entity, or a detectable label.

DNA dendrimers have been used for targeted delivery of a variety of cargo molecules to cells, including diagnostic and therapeutic cargos. The ability to derivatize DNA dendrimers with combinations of bioactive and targeting moieties allows for delivery of functionally-retentive materials to specific cell types following systemic administration. RNA interference (RNAi) is the process by which expression of a target gene is effectively silenced by selective inactivation of its corresponding mRNA as a consequence of directed generation of double-stranded RNA (dsRNA). Among the bioactive molecules that can be linked to DNA dendrimers are RNAs that promote dsRNA-induced gene silencing by cellular processes that degrade RNA, such as siRNA (small interfering RNA). The gene silencing mechanism of siRNA is degradation of the target mRNA, thus decreasing the amount of translated protein. siRNAs are double-stranded RNAs typically 21-23 nucleotides long that activate RNAi and lead to the degradation of mRNAs in a sequence-specific manner dependent upon complimentary binding to the target mRNA.

There continues to be a need for improved compositions and methods for treatment of cancer, including improved targeted delivery of effective therapeutic agents to cancer cells. The present disclosure is directed to these needs.

### SUMMARY

One aspect of the present invention is related to compositions comprising a DNA dendrimer linked to both a siRNA which inhibits expression of a cancer-associated mRNA by a cancer cell and a moiety which specifically binds to a cell surface receptor or cell surface protein on the cancer cell (the "targeting moiety") which is internalized by the cell upon binding to the targeting moiety. In certain embodiments, the targeting moiety may specifically bind to a cell surface receptor or cell surface protein which is over-expressed on the cancer cell as compared to non-cancerous cells.

In a first embodiment of the compositions, the siRNA linked to the DNA dendrimer may be a siRNA which inhibits expression of HuR. Specific non-limiting examples of such siRNAs include the following double-stranded siRNAs with 3 'overhangs:
mouse (m)HuR siRNA -
   5'- GCCUGUUCAGCAGCAUUGGdTdT-3' (SEQ ID NO:1)
   3'- dTdTCGGACAAGUCGUCGUAACC-5' (SEQ ID NO:3)
   and
human (h)HuR siRNA-
   5'- GCGUUUAUCCGGUUUGACAdTdT-3' (SEQ ID NO:2)
   3'- aaCGCAAAUAGGCCAAACUGU-5' (SEQ ID NO:4)
The upper strand is the sense strand and the lower strand is the antisense strand in the siRNAs SEQ ID NO:1/SEQ ID NO:3 and SEQ ID NO:2/SEQ ID NO:4. Lowercase "a" in SEQ ID NO:4 indicates a modification that forms a blunt end at the 3' end which allows the double-stranded siRNA to be processed by Dicer into a siRNA with symmetric 2-base 3' overhangs on each end. These overhangs result in preferred use of the antisense strand by RISC.

In a second embodiment of the compositions, the targeting moiety linked to the DNA dendrimer may be selected from the group consisting of folate (targeting folate receptors which are over-expressed on 90% of ovarian cancers), transferrin (targeting the transferrin receptor (TfR) which is over-expressed on many human cancers) and anti-mesothelin antibodies or antibody fragments (targeting mesothelin which is over-expressed on mesothelioma and many pancreatic and ovarian cancers). In a further specific example, the targeting moiety linked to the DNA dendrimer may be folate.

In a third specific embodiment the DNA dendrimer may be linked to folate and to a siRNA selected from the group consisting of SEQ ID NO:1/SEQ ID NO:3 and SEQ ID NO:2/SEQ ID NO:4. In a specific example, the DNA dendrimer may be linked to folate and to siRNA SEQ ID NO:2/SEQ ID NO:4.

It will be understood that embodiments of the compositions listed above may be combined not only as specifically described, but in other combinations suitable for a particular use. That is, any siRNA appropriate for inhibition of expression of one or more selected mRNAs in cancer cells of a particular type may be linked to the DNA dendrimer with any targeting moiety that allows binding of the composition to a cell surface protein or receptor that is over-expressed on the cancer cell as compared to non-cancerous cells.

In other embodiments, any of the foregoing compositions may be further modified and derivatized to improve stability of the DNA dendrimer, the siRNA, or both. Specific non-limiting examples include modification of the DNA dendrimer and/or the siRNA by attachment of polyethylene glycol (PEG), a fluorescent dye such as fluorescein or a fluorescein derivative, biotin or a biotin derivative, or digoxigenin to increase circulation time and efficacy of the composition. A specific non-limiting example of a modification that stabilizes the siRNA includes 2'-methoxy modification (also referred to as O-methyl modification) of one or more nucleotides at the 5' end of the antisense strand.

In other embodiments, any of the foregoing compositions may further comprise a therapeutic molecule in addition to the siRNA. The additional therapeutic molecule may be non-covalently or covalently linked to the DNA dendrimer by any of the attachment means described herein for attachment of the targeting moiety and the siRNA. In certain embodiments the additional therapeutic molecule will be linked to an arm of the DNA dendrimer which is not occupied by either the targeting moiety or the siRNA. Specific non-limiting examples of additional therapeutic molecules include nucleic acid therapeutics, antibody therapeutics and small molecule therapeutics. In specific non-limiting examples the additional therapeutic molecule may be a chemotherapeutic agent. In further non-limiting examples, the chemotherapeutic agent may be a cancer chemotherapeutic agent intercalated in the double-stranded portion of the DNA carrier, such as doxorubicin, daunomycin, daunorubicin, dactinomycin, cisplatin, carboplatin or voreloxin.

Another aspect of the invention relates to pharmaceutical formulations comprising the compositions described above. In certain embodiments, the DNA dendrimer linked to both a targeting moiety and a siRNA which inhibits expression of a cancer-associated mRNA by a cancer cell may be formulated with one or more pharmaceutically acceptable excipients. In a modification of any of these embodiments, the pharmaceutical formulation may be adapted for parenteral administration, such as injection or infusion.

Another aspect of the present disclosure is related to methods of using the compositions discussed above for inhibiting proliferation of tumor cells which overexpress HuR and/or inhibiting growth of tumors which overexpress HuR. In certain examples, inhibition of tumor cell proliferation and/or inhibition of tumor growth include methods for treating HuR-overexpressing cancer.

In a first embodiment of such methods, a composition according to any of the foregoing embodiments and examples may be administered to cancer cells *in vitro* or to a cancer patient *in vivo* in an amount effective to inhibit tumor cell proliferation, inhibit tumor growth, and/or treat the cancer in the patient.

In a second embodiment of the methods, the composition according to any of the foregoing embodiments and examples may be administered to a cancer patient with ovarian cancer, pancreatic cancer, breast cancer, lung cancer or mesothelioma in an amount effective to inhibit tumor cell proliferation, inhibit tumor growth and/or treat the cancer. In a specific example, the composition according to any of the foregoing embodiments and examples may be administered to a patient with ovarian cancer in an amount effective to inhibit proliferation of ovarian tumor cells, inhibit ovarian tumor growth, and/or treat the ovarian cancer in the patient. In a further specific example, the composition administered may be a DNA dendrimer linked to folate and to a siRNA selected from the group consisting of SEQ ID NO:1/SEQ ID NO:3 and SEQ ID NO:2/SEQ ID NO:4, wherein the composition may be administered in an amount effective to inhibit proliferation of ovarian tumor cells, inhibit ovarian tumor growth, and/or treat ovarian cancer in the patient. In a further specific example, the targeting moiety linked to the DNA dendrimer may be folate and the siRNA linked to the DNA dendrimer may be SEQ ID NO:2/SEQ ID NO:4.

In other embodiments, any of the methods of using the compositions and pharmaceutical formulations described herein may further comprise administration of a therapeutic agent in addition to the DNA dendrimer linked to both a siRNA which inhibits expression of a cancer-associated mRNA and a targeting moiety. In specific non-limiting examples, any of the additional therapeutic agents described above for linking to the DNA dendrimer may also be administered separately as additional therapeutic agents.

The above disclosure relating to methods of medical treatment are intended to include any of the DNA dendrimer compositions and pharmaceutical formulations herein disclosed for use as a medicament and/or for use in the treatment of cancer. In certain embodiments, included are the DNA dendrimer compositions and pharmaceutical formulations for use in the treatment of cancers which overexpress HuR. In other embodiments, included are the DNA dendrimer compositions and pharmaceutical formulations for use in the treatment of ovarian cancer.

### BRIEF DESCRIPTION OF THE DRAWINGS

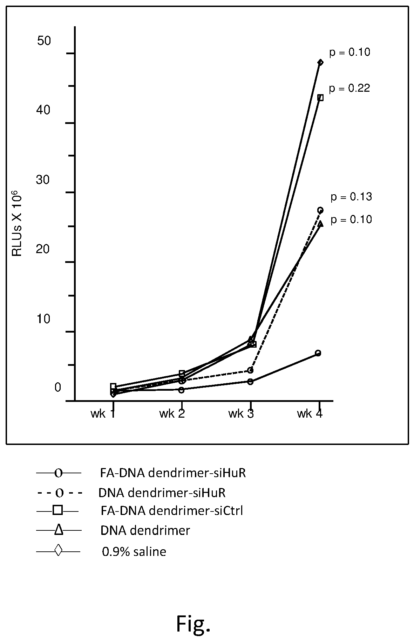

The Figure illustrates the effect of treatment with siHuR on ovarian cancer cell growth.

### DETAILED DESCRIPTION

Reference throughout this specification to "one embodiment," "certain embodiments," "various embodiments," "one or more embodiments" or "an embodiment" means that a particular feature, structure, material, or characteristic described in connection with the embodiment is included in at least one embodiment of the invention. Thus, the appearances of the phrases such as "in one or more embodiments," "in certain embodiments," "in various embodiments," "in one embodiment" or "in an embodiment" in various places throughout this specification are not necessarily referring to the same embodiment of the invention. Furthermore, the particular features, structures, materials, or characteristics may be combined in any suitable manner in one or more embodiments.

As used herein, the term "linked to a DNA dendrimer" and its equivalents refer to attachment of a non-dendrimer moiety to an arm of the DNA dendrimer by covalent or noncovalent binding. The linkage may involve formation of a chemical bond between the arm of the DNA dendrimer and the non-dendrimer moiety, or linkage may occur through hybridization of a nucleic acid attached to the non-dendrimer moiety to a complementary nucleic acid sequence on the dendrimer arm. Such hybridization bonding may be directly between the sequence on the non-dendrimer moiety and the sequence of the dendrimer arm, or it may be indirect hybridization in which a capture sequence hybridized to both the dendrimer arm and the sequence attached to the non-dendrimer moiety forms a hybridization "bridge" between the two.

As used herein, the term "inhibition of expression" and its equivalents refer to reduction in the level of mRNA transcribed from a gene. The reduction in mRNA may be either due to a lower level of transcription or to an increase in post-transcriptional destruction of the mRNA.

As used herein, the term "cancer-associated mRNA" and its equivalents refer to an mRNA that is increased in cancer cells as compared to normal cells. The increase in the mRNA may be due to an increased rate of transcription in cancer cells as compared to normal cells, amplification of a gene, or a reduction in post-transcriptional degradation of the mRNA resulting in longer mRNA half-life.

As used herein, the term "targeting moiety" and its equivalents refer to a molecule which recognizes and binds to a complementary molecule on the surface of a cell or tissue. Non-limiting examples of targeting moieties include antibodies, antibody fragments, binding proteins and peptides, receptors and ligands for receptors. A specific binding partner for the targeting moiety means that the targeting moiety does not bind substantially to other binding partners. In some cases the targeting moiety/receptor complex or the targeting moiety/specific binding partner complex may be internalized by the cell.

As used herein, the terms "inhibition of tumor cell proliferation," "inhibition of tumor growth" and their equivalents refer to slowing of the overall rate of cell division in the tumor (which does not necessarily mean slowing of the rate of cell division in every cell in the tumor) with treatment such that the overall increase in tumor size over time is reduced compared to an increase in tumor size without treatment.

As used herein, the term "treatment of cancer" and its equivalents refer to inhibiting growth or spread of cancer, alleviating the symptoms of cancer, and/or substantially eliminating cancer from a patient.

Compositions comprising a DNA dendrimer linked to both a siRNA which inhibits expression of a cancer-associated mRNA and a targeting moiety are one aspect of the present invention. In general, the targeting moiety will specifically bind to a cell surface receptor or a cell surface protein on a cancer cell to deliver the siRNA to the targeted cancer cell. Any cell surface receptor or cell surface protein may be utilized for binding the targeting moiety of the DNA dendrimer composition. Use of targeting moieties that bind to cell surface receptors or cell surface proteins which are internalized after binding to the targeting moiety are advantageous to deliver the siRNA into the cell where it can more effectively degrade the complementary mRNA. Use of targeting moieties that bind to cell surface receptors and cell surface proteins that are over-expressed on the cancer cell are advantageous to provide improved specificity of siRNA action on degradation of the complementary mRNA in cancer cells as compared to normal cells which may also express the cell surface receptor or cell surface protein, albeit at lower amounts than on tumor cells.

The targeting moiety may be linked to an available arm of the DNA dendrimer by any of several known chemistries depending on the chemical nature of the targeting moiety. Examples include formation of a disulfide bridging bond, by *N*-hydroxysuccinimide ester dependent condensation, by use of a bifunctional cross-linking reaction, by direct or indirect hybridization of the targeting moiety to the arm of the DNA dendrimer, or by use of polycationic compounds to bridge the targeting moiety to the DNA dendrimer via charge-charge interactions. These linking reactions are described in WO 2010/017544 with respect to RNA, but may be applied to other appropriate targeting moieties. A nucleic acid targeting moiety may also be linked to the DNA dendrimer by blunt-end ligation using a bridging oligonucleotide as described in U.S. Patent No. 8685899. Appropriate options for hybridization of the targeting moiety to the DNA dendrimer include appending a nucleic acid capture sequence to the targeting moiety which is complementary to a sequence at the end of the arm of the DNA dendrimer, as described in WO 02/33125.

The siRNA will generally be linked to a different available arm of the DNA dendrimer than the targeting moiety. The siRNA may be linked to an available arm of the DNA dendrimer by formation of a disulfide bridging bond, by *N*-hydroxysuccinimide ester dependent condensation, by use of a bifunctional cross-linking reaction, by direct or indirect hybridization of the siRNA to the arm of the DNA dendrimer, or by use of polycationic compounds to bridge the siRNA to the DNA dendrimer via charge-charge interactions. These linking reactions are described in WO 2010/017544. The siRNA may also be linked to the DNA dendrimer by blunt-end ligation using a bridging oligonucleotide as described in U.S. Patent No. 8685899. Appropriate options for hybridization of the siRNA to the DNA dendrimer include appending a capture sequence to the RNA which is complementary to a sequence at the end of the arm of the DNA dendrimer, as described in WO 02/33125.

Any targeting moiety which binds to a cell surface receptor or cell surface protein on a cell type of interest may be linked to the DNA dendrimer. For anti-cancer therapeutic uses of the DNA dendrimer compositions, it will generally be desirable to select a targeting moiety which binds to a cell surface receptor or cell surface protein specifically expressed on cancer cells or over-expressed on cancer cells. Examples of such targeting moieties include folate, transferrin, and anti-mesothelin antibodies, among other suitable targeting moieties known to the person skilled in the art.

Any siRNA which hybridizes to and degrades a complementary cancer-associated mRNA expressed in a cancer cell of interest may be linked to the DNA dendrimer. Non-limiting examples of such mRNAs include HuR, which is over-expressed in ovarian cancer cells. Any siRNA which binds to HuR mRNA and results in its degradation is appropriate for use in the DNA dendrimer compositions. Specific non-limiting examples of useful siRNAs that may be linked to the DNA dendrimer along with the targeting moiety include SEQ ID NO:1/SEQ ID NO:3 and SEQ ID NO:2/SEQ ID NO:4. In further non-limiting examples, siRNAs SEQ ID NO:1/SEQ ID NO:3 or SEQ ID NO:2/SEQ ID NO:4 may be linked to the DNA dendrimer with folate linked to the DNA dendrimer as the targeting moiety.

In any of the foregoing DNA dendrimer compositions, the linkage of the targeting moiety and the siRNA to the DNA dendrimer may be independently selected from the group consisting of direct linkage to the arms of the DNA dendrimer via chemical conjugation and hybridization between a capture oligonucleotide ligated to the arm of the DNA dendrimer and a complementary carrier oligonucleotide linked to the targeting moiety and/or siRNA.

In specific embodiments of any of the foregoing DNA dendrimer compositions, the targeting moieties and/or the siRNAs may be linked to the DNA dendrimer via a capture oligonucleotide associated with the arm of the DNA dendrimer. The capture oligonucleotide is generally associated with the terminus of the DNA dendrimer arm. Typically it is ligated to the terminus of the arm of the DNA dendrimer, but it may also be hybridized to the terminus and optionally crosslinked thereto or associated with the arm by use of an extension oligonucleotide as described below. The capture oligonucleotide provides a specific, defined sequence present in a defined quantity for hybridization to a complementary carrier oligonucleotide linked to the targeting moiety and/or the siRNA. The capture oligonucleotide also provides a means for controlling the number of targeting moieties and siRNAs linked to the DNA dendrimer, as the carrier oligonucleotides can be hybridized to the capture oligonucleotide at a defined concentration which results in the desired number of DNA dendrimer arms being occupied by each component. Upon hybridization of the complementary carrier oligonucleotide, the targeting moiety and/or siRNA becomes linked to the arm of the DNA dendrimer through Watson-Crick base pairing.

Any of the foregoing DNA dendrimer compositions may be formulated as pharmaceutical formulations with pharmaceutically acceptable excipients. Such excipients may include one or more component selected from the group consisting of a solvent, a dispersing agent, a coating (e.g., lecithin), a surfactant (e.g., hydroxypropylcellulose), a preservative (e.g., paraben, phenol, thimerosal, sorbic acid, chlorobutanol), an emulsion, an alcohol (e.g., ethanol), a polyol (e.g., glycerol, propylene glycol), and an isotonic agent (e.g., sugars, sodium chloride).

The pharmaceutical formulations comprising the DNA dendrimer compositions may also be adapted for specific routes of administration as is known in the art. As non-limiting examples, the pharmaceutical formulations may be formulated as solutions for injection or infusion, or as tablets for oral administration. Solutions or dispersions of the DNA dendrimer compositions may be prepared, for example, in water or saline.

Pharmaceutical formulations comprising the DNA dendrimer compositions are useful for treatment of cancer in a patient, inhibiting tumor cell proliferation and/or inhibiting tumor growth. Methods for inhibiting tumor cell proliferation and/or inhibiting tumor growth may be applied either *in vivo,* for treatment of cancer patients, or *in vitro,* such as for research or diagnostic purposes. In general, the amount of the pharmaceutical formulation administered to a cancer patient *in vivo* will be an amount effective to inhibit tumor cell proliferation, inhibit tumor growth, and/or treat the cancer in the patient. The effective amount will be based on a combination of the dose administered, the duration of administration, the administration protocol, and the route of administration. One skilled in the art will recognize that individualization of dosage based on a patient's body composition or his/her response to treatment may be medically necessary or desirable.

Methods for treatment of cancer in a patient may include administration to the subject by parenteral, intraperitoneal, intravenous, intratumoral, or oral routes. Particularly useful routes of administration include injection, infusion, or oral administration. For treatment of cancer, a typical course of administration may include IV administration continuously or intermittently over a course of several weeks, often followed by a period of no administration and then at least one repetition of the intermittent administration schedule.

### EXAMPLES

In Vitro Assays for siRNA Stability, Functionality and Biodistribution:

siHuR stability was evaluated in PBS, serum free medium and medium +10% serum. 10% TBU PAGE of siHuR showed that the sense and antisense strands were not degraded in any of the samples.

For evaluation of functionality, 2-layer DNA dendrimers linked to folate, the siHuR sequence and a fluorescent label (Cy3) in various combinations were prepared. siRNA stability was tested by transfecting cells with the following: siHuR + Lipofectamine, DNA dendrimer linked to folate, and DNA dendrimer linked to both folate and siHuR. Controls were untransfected cells and Lipofectamine only.

siHuR (SEQ ID NO:2/SEQ ID NO:4) was combined with either lipofectamine or the targeted DNA dendrimer and incubated overnight with A2780 cells (a human ovarian tumor cell line) in a medium containing 10% serum. HuR mRNA expression in each case was compared to untransfected cells (100% HuR expression). Results are shown in the following

**Table:**

| | Untransfected Cells | Lipofectamine only | Lipofectamine +siHuR | Targeted Dendrimer only | Targeted Dendrimer +siHuR |
|---|---|---|---|---|---|
| %HuR Expression | 100 | 96 | 35.4 | 94 | 26.8 |

Targeting of siHuR to ovarian cancer cells using folate linked to a DNA dendrimer significantly improved HuR mRNA knock-down compared to transfection of siHuR alone.

A biodistribution study in ovarian tumor bearing mice done with systemically administered DNA dendrimer linked to CY3 and to folate showed very high fluorescence in ovarian tumors with little to no fluorescence in normal tissues (brain, liver, spleen, lung, heart and normal ovary). These results suggest that specificity of cancer treatment may be achieved using folate as the targeting moiety linked to the DNA dendrimer for cancers that over-express the folate receptor.

In Vivo Testing for Inhibition of Ovarian Tumor Growth: Ovarian tumor bearing mice were injected twice per week intraperitoneally with 2 µg siHuR (SEQ ID NO:1/SEQ ID NO:3) as: DNA dendrimer linked to folate and to siHuR, DNA dendrimer linked to siHuR, and DNA dendrimer linked to folate and a negative control siRNA. Controls were underivatized DNA dendrimer and 0.9% saline (untreated). Injections were continued for four weeks.

The model system was C57BL/6 female mice injected i.p. with ID8-Fluc murine ovarian cancer cells, six mice per treatment group. Tumors developed throughout the peritoneal cavity. Approximately 4 weeks following injection, tumor-bearing mice were optically imaged to establish baseline bioluminescence, an indicator of ID8-luc-derived tumor load, then were treated twice a week for four weeks (retro-orbital injection) with one of four DNA dendrimer formulations or with 0.9% saline (n = 5-6/group). The four DNA dendrimer formulations were: 1) DNA dendrimer linked to folate and to siHuR, 2) DNA dendrimer linked to siHuR; 3) DNA dendrimer linked to folate and to a negative control siRNA; and 4) underivatized DNA dendrimer. Tumor response to treatment was assessed once a week by optical imaging. Results are shown in the Figure. Using baseline tumor load as the comparator, tumor growth was suppressed -3-fold in mice treated with DNA dendrimer linked to folate and to siHuR for 4 weeks. This was the highest suppression of all treatment groups, including DNA dendrimer linked to siHuR (p = 0.13), and it was substantially better than in mice treated with DNA dendrimer linked to folate and negative control siRNA (p = 0.22), underivatized DNA dendrimer (p = 0.10), or 0.9% saline (p = 0.10). These results support the enhanced therapeutic effect that may be observed for treatment of cancer using DNA dendrimer linked to folate and to siHuR.

## Claims

1. A DNA dendrimer linked to a targeting moiety and to a siRNA which inhibits expression of HuR, wherein the siRNA is selected from the group consisting of SEQ ID NO:1/SEQ ID NO:3 and SEQ ID NO:2/SEQ ID NO:4.

2. The DNA dendrimer according to claim 1, wherein the targeting moiety is selected from the group consisting of folate, transferrin, anti-mesothelin antibody, and combinations thereof.

3. The DNA dendrimer according to claim 2, wherein the targeting moiety is folate and the siRNA is SEQ ID NO:2/SEQ ID NO:4.

4. A pharmaceutical composition comprising a DNA dendrimer according to claim 1 and at least one pharmaceutically acceptable excipient.

5. The pharmaceutical composition according to claim 4, wherein the targeting moiety is folate and the siRNA is SEQ ID NO:2/SEQ ID NO:4.

6. The pharmaceutical composition according to claim 4, which is formulated for injection or infusion.

7. A composition comprising a DNA dendrimer according to any one of Claims 1-3 for use in inhibiting proliferation of tumor cells which overexpress HuR and/or inhibiting growth of a tumor which overexpresses HuR, the use comprising administering to the tumor cells or tumor the composition comprising a DNA dendrimer linked to a targeting moiety and to a siRNA which inhibits expression of HuR in an amount effective to inhibit tumor cell proliferation, inhibit tumor growth and/or treat cancer.

8. A composition comprising a DNA dendrimer according to any one of Claims 1-3 for use according to claim 7, wherein the use comprises administration of the composition to a patient with ovarian cancer.

9. A composition comprising a DNA dendrimer according to any one of Claims 1-3 for use according to claim 7, wherein the use comprises administration of the composition by injection or infusion.

10. A DNA dendrimer according to any one of Claims 1-3 for use as a medicament.

## Patentansprüche

1. DNA-Dendrimer, das an eine Zielgruppierung und an eine siRNA, welche eine Expression von HuR hemmt, gebunden ist, wobei die siRNA aus der Gruppe bestehend aus SEQ ID NO:1/SEQ ID NO:3 und SEQ ID NO:2/SEQ ID NO:4 ausgewählt ist.

2. DNA-Dendrimer nach Anspruch 1, wobei die Zielgruppierung aus der Gruppe bestehend aus Folat, Transferrin, Antimesothelin-Antikörper und Kombinationen davon ausgewählt ist.

3. DNA-Dendrimer nach Anspruch 2, wobei die Zielgruppierung Folat ist und die siRNA SEQ ID NO:2/SEQ ID NO:4 ist.

4. Pharmazeutische Zusammensetzung, umfassend ein DNA-Dendrimer nach Anspruch 1 und mindestens einen pharmazeutisch verträglichen Exzipienten.

5. Pharmazeutische Zusammensetzung nach Anspruch 4, wobei die Zielgruppierung Folat ist und die siRNA SEQ ID NO:2/SEQ ID NO:4 ist.

6. Pharmazeutische Zusammensetzung nach Anspruch 4, die zur Injektion oder Infusion formuliert ist.

7. Zusammensetzung, umfassend ein DNA-Dendrimer nach einem der Ansprüche 1-3 zur Verwendung beim Hemmen einer Proliferation von Tumorzellen, die HuR überexprimieren, und/oder beim Hemmen eines Wachstums eines Tumors, der HuR überexprimiert, wobei die Verwendung ein Verabreichen der Zusammensetzung, die ein DNA-Dendrimer umfasst, das an eine Zielgruppierung und an eine siRNA, welche eine Expression von HuR hemmt, gebunden ist, in einer Menge an die Tumorzellen oder den Tumor umfasst, die wirksam ist, eine Tumorzellproliferation zu hemmen, ein Tumorwachstum zu hemmen und/oder Krebs zu behandeln.

8. Zusammensetzung, umfassend ein DNA-Dendrimer nach einem der Ansprüche 1-3 zur Verwendung nach Anspruch 7, wobei die Verwendung eine Verabreichung der Zusammensetzung an eine Patientin mit Eierstockkrebs umfasst.

9. Zusammensetzung, umfassend ein DNA-Dendrimer nach einem der Ansprüche 1-3 zur Verwendung nach Anspruch 7, wobei die Verwendung eine Verabreichung der Zusammensetzung durch Injektion oder Infusion umfasst.

10. DNA-Dendrimer nach einem der Ansprüche 1-3 zur Verwendung als Medikament.

## Revendications

1. Dendrimère d'ADN lié à une fraction de ciblage et à un ARNsi qui inhibe l'expression du HuR, dans lequel l'ARNsi est choisi dans le groupe constitué par la SEQ ID NO: 1/SEQ ID NO: 3 et la SEQ ID NO: 2/SEQ ID NO: 4.

2. Dendrimère d'ADN selon la revendication 1, dans lequel la fraction de ciblage est choisie dans le groupe constitué du folate, de la transferrine, de l'anticorps anti-mésothéline et de leurs combinaisons.

3. Dendrimère d'ADN selon la revendication 2, dans lequel la fraction de ciblage est le folate et l'ARNsi est la SEQ ID NO: 2/SEQ ID NO: 4.

4. Composition pharmaceutique comprenant un dendrimère d'ADN selon la revendication 1 et au moins un excipient pharmaceutiquement acceptable.

5. Composition pharmaceutique selon la revendication 4, dans laquelle la fraction de ciblage est le folate et l'ARNsi est la SEQ ID NO: 2/SEQ ID NO: 4.

6. Composition pharmaceutique selon la revendication 4, qui est formulée pour une injection ou une perfusion.

7. Composition comprenant un dendrimère d'ADN selon l'une quelconque des revendications 1 à 3 pour une utilisation dans l'inhibition de la prolifération de cellules tumorales qui surexpriment le HuR et/ou l'inhibition de la croissance d'une tumeur qui surexprime le HuR, l'utilisation comprenant l'administration aux cellules tumorales ou à la tumeur de la composition comprenant un dendrimère d'ADN lié à une fraction de ciblage et à un ARNsi qui inhibe l'expression du HuR en une quantité efficace pour inhiber la prolifération des cellules tumorales, inhiber la croissance tumorale et/ou traiter le cancer.

8. Composition comprenant un dendrimère d'ADN selon l'une quelconque des revendications 1 à 3 pour une utilisation selon la revendication 7, dans laquelle l'utilisation comprend l'administration de la composition à un patient atteint d'un cancer de l'ovaire.

9. Composition comprenant un dendrimère d'ADN selon l'une quelconque des revendications 1 à 3 pour une utilisation selon la revendication 7, dans laquelle l'utilisation comprend l'administration de la composition par injection ou perfusion.

10. Dendrimère d'ADN selon l'une quelconque des revendications 1 à 3 pour une utilisation en tant que médicament.
